(19) 

Europäisches
Patentamt
European
Patent Office
Office européen
des brevets

(11) **EP 4 647 061 A1**

(12) **EUROPEAN PATENT APPLICATION**

(43) Date of publication:
**12.11.2025 Bulletin 2025/46**

(21) Application number: **24220383.4**

(22) Date of filing: **16.12.2024**

(51) International Patent Classification (IPC):
**A61K 8/11** *(2006.01)* **A61K 8/64** *(2006.01)*
**A61K 8/9789** *(2017.01)* **A61Q 19/08** *(2006.01)*
**A61K 8/67** *(2006.01)*

(52) Cooperative Patent Classification (CPC):
**A61K 8/671; A61K 8/11; A61K 8/64; A61K 8/9789;
A61Q 19/08**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.05.2024 CN 202410574649**

(71) Applicant: **Guangzhou Jiyan Cosmetics
Technology Co., Ltd.
Guangzhou, Guangdong Province 510000 (CN)**

(72) Inventors:
• **WANG, Mengping
Guangzhou, 510000 (CN)**

• **LU, Wangwang
Guangzhou, 510000 (CN)**
• **CHENG, Jing
Guangzhou, 510000 (CN)**
• **ZHANG, Yi
Guangzhou, 510000 (CN)**
• **CAI, Zhuo
Guangzhou, 510000 (CN)**
• **WANG, Bingjie
Guangzhou, 510000 (CN)**
• **YU, Pengyong
Guangzhou, 510000 (CN)**
• **GARNIER, Mathilde
31000 Toulouse (FR)**

(74) Representative: **Vidon Brevets & Stratégie
16B, rue de Jouanet
35700 Rennes (FR)**

(54) **COMPOSITION CONTAINING RETINOL AND SYNERGIST AND USE THEREOF**

(57) The present disclosure relates to the field of cosmetics, and particularly to a composition and use thereof. The present disclosure provides a composition, including encapsulated retinol and a synergist, wherein the synergist includes one or more of tetrapeptide-1 or *pistacia lentiscus* gum; and a mass ratio of the encapsulated retinol to the synergist is 2-3:1-2. Experimental results show that the tetrapeptide-1, as a synergist of encapsulated retinol, can remarkably improve transcription of type I collagen and type III collagen, thereby improving tightening and anti-wrinkle effects; the pistacia lentiscus gum, as a synergist of encapsulated retinol, can remarkably improve transcription of type I collagen, thereby improving anti-aging effect of retinol. In addition, addition of the tetrapeptide-1 and pistacia lentiscus gum can also enhance up-regulation level of the encapsulated retinol to cell CRABP2, thereby improving intracellular transport efficiency of retinoic acid (an active form of retinol), and better exerting its signal regulating effect.

**EP 4 647 061 A1**

**Description**

**TECHNICAL FIELD**

The present disclosure relates to the field of cosmetics, and particularly to a composition containing retinol and a synergist and use thereof.

**BACKGROUND ART**

Retinol, as a classical anti-aging component in the field of cosmetics, has widely known efficacies such as improving fine lines and resisting photoaging because it can be converted into retinoic acid *in vivo,* then bind to retinoic acid binding protein, enter nucleus to regulate and control multiple genes (collagen, elastin, matrix metalloproteinase, cell differentiation, etc.).

However, retinol contains a plurality of conjugated double bonds in molecular structure, and thus is unstable, sensitive to high temperature, sunlight and oxygen, and is susceptible to denaturation and inactivation. Many material developers improve stability of retinol raw materials by modifying retinol molecule or encapsulating retinol, such as retinyl palmitate, retinyl propionate or liposome-encapsulated retinol. The above modes of treating retinol, although improving the stability of retinol, reduces an effective dose thereof to some extent.

Therefore, how to efficiently and stably apply retinol to cosmetics has always been a major problem that troubles experts and engineers in the industry.

**SUMMARY**

In view of this, the present disclosure provides a composition and use thereof. In the composition provided by the present disclosure, retinol has good stability, and efficacy of retinol can be improved.

The present disclosure provides a composition, including encapsulated retinol and a synergist.

The synergist includes one or more of tetrapeptide-1 or *pistacia lentiscus* gum.

A mass ratio of the encapsulated retinol to the synergist is 2-3:1-2.

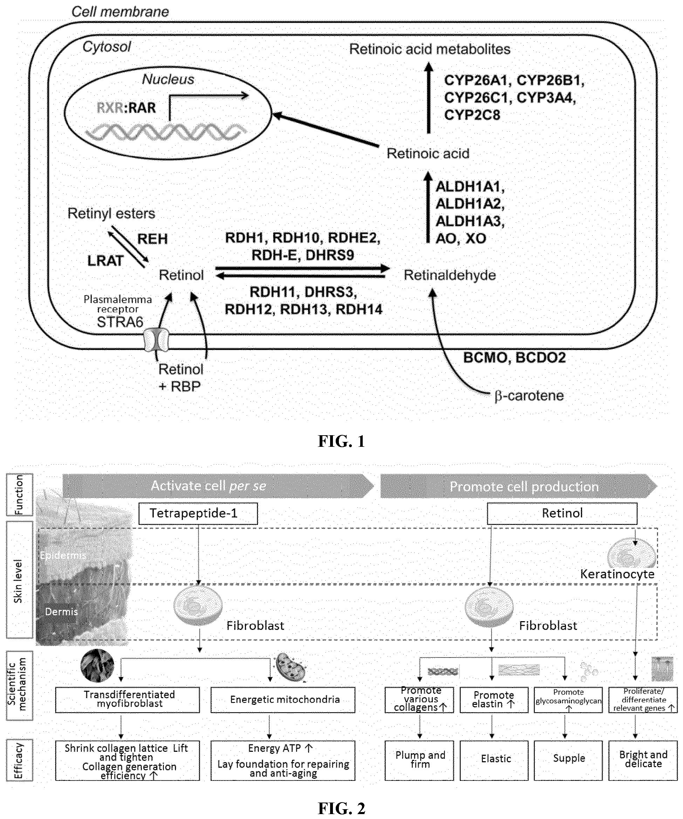

It is well-known that to exert efficacy in skin cells, retinol needs to be transported to target cell via binding protein, converted into retinoic acid (an active form of retinol), and then transported to nucleus via binding protein, to activate target gene. A specific process is as shown in FIG. 1, which shows a process of enzyme regulating steady state of human retinoic acid. However, retinoic acid (an active form of retinol) has a very short half-life and is readily degraded and inactivated by cytochrome P450 enzyme (CYP 26s enzyme). Therefore, to make retinol and other VA (abbreviation of retinoids) substances exert lasting efficacy, a synergist is needed to improve its activity of binding protein or inhibit its vitality of degrading enzyme to some extent. This is of great significance for retinol and other VA substances to exert efficacy, but is ignored by many people. The inventors found a plant material (*pistacia lentiscus* gum) that can inhibit CYP26s enzyme activity and improve endogenous VA level of skin, and is speculated to have the potential for synergizing VA.

Furthermore, in the aging process, extracellular matrix loss/damage is the most direct cause, but a cell's own state is reduced, and a key signaling pathway is inhibited, all of which are potential core causes. The inventors found tetrapeptide-1 that has an innovative mechanism, can stimulate transdifferentiation of fibroblast into myofibroblast, and promote the same to express α-smooth muscle actin. The tetrapeptide-1, on the one hand, can enhance its contractility to collagen network and provide stronger supporting performance, and on the other hand, can also promote generation of more extracellular matrix (such as collagen). Due to such transdifferentiation stimulation to cell, the tetrapeptide-1 is quite suitable as a synergist for retinoids such as retinol, and is of important significance for improving looseness and wrinkles during aging. As shown in FIG. 2, FIG. 2 shows synergy mechanism of the tetrapeptide-1 for retinol.

In the present disclosure, the tetrapeptide-1 or *pistacia lentiscus* gum is used as a synergist for encapsulated retinol. The encapsulated retinol *per se* has relatively good stability. The synergist can enhance efficacy of retinol in skin, and improve tightening and anti-wrinkle effects of retinol by activating cell and regulating and controlling effect of biological enzyme. Experimental results show that the tetrapeptide-1, as a synergist of encapsulated retinol, can remarkably improve transcription of type I collagen and type III collagen, thereby improving the tightening and anti-wrinkle effects; the *pistacia lentiscus* gum, as a synergist of encapsulated retinol, can remarkably improve transcription of cellular retinoic acid binding protein 2 (CRABP2), thereby improving intracellular transport efficiency of retinoic acid (an active form of retinol), and better exerting its signal regulating effect.

In the present disclosure, encapsulated retinol is used as a raw material, that is, an encapsulating substance is formed on a surface of retinol, which can improve the stability of retinol. In some specific implementations, a content of retinol in the encapsulated retinol is preferably 5-20wt%, and more preferably 6-15wt%. In some specific implementations, the encapsulated retinol includes retinol and a shell encapsulating the retinol, where the shell is formed from cellulose acetate butyrate, tricaprylin, pentaerythrityl tetra-di-t-butyl hydroxyhydrocinnamate, silica silylate, silica and magnesium stearate. Specifically, the shell is formed from 73%-76% of cellulose acetate butyrate, 11%-13% of tricaprylin, 2%-4% of pentaerythrityl tetra-di-t-butyl hydroxyhydrocinnamate, 0.7%-1.3% of silica silylate, 0.4%-0.8% of silica and 0.2%-0.6% of magnesium stearate. In some specific implementations, trade name of the encapsulated retinol is CelluCap RL, and in the encapsulated retinol, a retinol content is about 9%, and an encapsulating material is cellulose acetate butyrate, tricaprylin, pentaerythrityl tetra-di-t-butyl hydroxyhydrocinnamate , silica silylate, silica and magnesium stearate.

In the present disclosure, the tetrapeptide-1 and *pistacia lentiscus* gum, as synergists for the encapsulated retinol, can improve the activity of retinol. In some specific implementations, trade name of the tetrapeptide-1 is Uplevity e-Lift peptide. In some specific implementations, trade name of the *pistacia lentiscus* gum is NovoRetin.

In some specific implementations, a mass ratio of the encapsulated retinol to the synergist is 1-5:1-2, preferably 1.5-4.5:1-2, more preferably 2-4:1, further preferably 2-3:1, and most preferably 2.78-2.88:1.

In some specific implementations, a mass ratio of the encapsulated retinol to the tetrapeptide-1 is 1-5:1, preferably 1.5-4.5:1, more preferably 2-3:1, more preferably 2.5-3:1, more preferably 2.7-3:1, further preferably 2.7-2.8:1, most preferably 2.78-2.88:1, and by far most preferably 2.8:1.

In some specific implementations, a mass ratio of the encapsulated retinol to the *pistacia lentiscus* gum is 1-5:2, preferably 1.5-4.5:2, more preferably 2-3:2, more preferably 2.5-3:2, more preferably 2.7-3:2, further preferably 2.7-2.8:2, most preferably 2.78-2.88:2, and by far most preferably 2.8:2.

In some specific implementations, when the encapsulated retinol is CelluCap RL, a mass ratio of the encapsulated retinol to the synergist is preferably 1-5:1, preferably 1.5-4.5:1, more preferably 2.5-3:1, and more preferably 2.7-3:1.

The present disclosure further provides use of the composition in the above technical solutions in preparation of anti-aging products.

In some specific implementations, the products include cosmetics.

The present disclosure further provides a product, including the composition in the above technical solutions, and the product is preferably a cosmetic.

In the present disclosure, the cosmetics can be classified into cleansing cosmetics, care cosmetics and makeup cosmetics according to cosmetic classification principle.

In the above, the cleansing cosmetics refer to cosmetics applied to a surface of human body (epidermis, hair, nails, lips, etc.) by means of smearing, spraying or other similar methods, to achieve a cleansing effect or eliminate unpleasant smell. The care cosmetics refer to cosmetics applied to a surface of human body (epidermis, hair, nails, lips, etc.) by means of smearing, spraying or other similar methods, to achieve a maintenance effect. The makeup cosmetics refer to cosmetics applied to a surface of human body (epidermis, hair, nails, lips, etc.) by means of smearing, spraying or other similar methods, to achieve effects of beautifying, decorating and increasing charm of human body.

The cleansing cosmetics suitable for skin include, but are not limited to, facial cleanser, make-up remover (cream), cleansing cream (emulsion), facial mask, toilet water, prickly-heat powder, talcum powder or bath fluid. The care cosmetics suitable for skin include, but are not limited to, skin care cream, emulsion or lotion. The makeup cosmetics suitable for skin include, but are not limited to, pressed powder, rouge, eye shadow, eyeliner (liquid eyeliner), eyebrow pencil, perfume or cologne. The cleansing cosmetics suitable for hair include, but are not limited to, shampoo, cream shampoo or shaving cream. The care cosmetics suitable for hair include, but are not limited to, hair conditioner, hair cream, hair oil/hair pomade or hot treatment conditioner. The makeup cosmetics suitable for hair include, but are not limited to, styling mousse/hair gel, hair dye, hair perming agent, mascara (cream), hair restorer or depilatory. The cleansing cosmetics suitable for nails include, but are not limited to, nail polish remover. The care cosmetics suitable for nails include, but are not limited to, nail care lotion (cream) and nail hardeners. The makeup cosmetics suitable for nails include, but are not limited to nail polish. The cleansing cosmetics suitable for lips include, but are not limited to, lip make-up remover. The care cosmetics suitable for lips include, but are not limited to, lip balm. The makeup cosmetics suitable for lips include, but are not limited to, lip stick, lip gloss or lip liner.

In the present disclosure, the tetrapeptide-1 or *pistacia lentiscus* gum is used as a synergist for encapsulated retinol. The encapsulated retinol *per se* has relatively good stability. The synergist can enhance efficacy of retinol in skin, and improve tightening and anti-wrinkle effects of retinol by activating cell and regulating and controlling effect of biological enzyme. Experimental results show that the tetrapeptide-1, as a synergist of encapsulated retinol, can remarkably improve transcription of type I collagen and type III collagen, thereby improving the tightening and anti-wrinkle effects; the *pistacia lentiscus* gum, as a synergist of encapsulated retinol, can remarkably improve transcription of cell CRABP2, thereby improving intracellular transport efficiency of retinoic acid (an active form of retinol), and better exerting its signal regulating effect.

## BRIEF DESCRIPTION OF DRAWINGS

FIG. 1 shows a process of enzyme regulating steady state of human retinoic acid;

FIG. 2 shows synergy mechanism of tetrapeptide-1 for retinol;

FIG. 3 shows changes in skin elasticity (A) and skin brightness L value (B) after subjects use cosmetics containing an encapsulated-retinol composition;

FIG. 4 shows photographs of faces of subjects taken by Visia.

## DETAILED DESCRIPTION OF EMBODIMENTS

It should be understood that the expression "one or more of..." separately includes each object described after the expression as well as various different combinations of two or more of the described objects, unless otherwise understood from the context and usage. The expression "and/or" in conjunction with three or more objects described should be understood to have the same meaning, unless otherwise understood from the context.

The terms "including", "having" or "containing", including use of grammatical equivalents thereof, should generally be understood as open-ended and non-limiting, for example, not excluding other unrecited elements or steps, unless specifically stated otherwise or otherwise understood from the context.

It should be understood that the order of steps or the order of performing certain actions is not important as long as the present disclosure still can be implemented. In addition, two or more steps or actions may be performed simultaneously.

The use of any and all examples or exemplary language such as "for example" or "including" herein is merely intended to better illustrate the present disclosure and does not constitute limitation to the scope of the present disclosure unless otherwise claimed. No language in the present description should be construed as indicating any non-claimed element as essential to the practice of the present disclosure.

In addition, numerical ranges and parameters used to define the present disclosure are all approximate numerical values, and relevant numerical values in specific examples have been presented herein as precisely as possible. However, any numerical values inherently inevitably contain standard deviations due to individual testing methods. Accordingly, unless otherwise expressly indicated, it should be understood that all ranges, amounts, numerical values and percentages used in the present disclosure are to be modified by "about". Herein, "about" generally means that an actual numerical value is a particular numerical value or within a range plus or minus 10%, 5%, 1% or 0.5%

The present disclosure will be further illustrated in conjunction with following examples.

In various examples below, trade name of encapsulated retinol is CelluCap RL, trade name of tetrapeptide-1 is Uplevity e-Lift peptide, and trade name of *pistacia lentiscus* gum (shorted as "gum" hereinafter) is NovoRetin.

## Test Example 1

Testing method: based on UVA-stimulated fibroblast model, transcription of type I and type III collagens was tested using qRT-PCR method, and specific steps were as follows:

1) Cell inoculation: after resuscitation of fibroblasts, when a plating rate reached about 60%, cells were inoculated in a 6-well plate, and incubated overnight in an incubator (37 °C, 5% $CO_2$).

2) Liquid formulation: according to test schemes (see TABLE 1 and TABLE 2), test substance working solutions were formulated respectively according to mass ratios.

TABLE 1 COL I Detection Scheme

| Group | Sample Name | Dosing Concentration | Stimulation Condition | Detection Model | Detection Item | Detection Method |
|---|---|---|---|---|---|---|
| Blank Control (BC) | / | / | No stimulation | Fibroblast | COLI (type I collagen) | qRT-PCR |
| Negative Control (NC) | / | / | UVA (30 J/cm$^2$) stimulation | | | |
| Positive Control (PC) | TGF-β1 | 100 ng/mL | | | | |
| Comparative Example 1 | Pro-xylane | 0.5000% | | | | |
| Comparative Example 2 | Tetrapeptide-1 | 0.00109% | | | | |
| Comparative Example 3 | Encapsulated retinol | 0.00313% | | | | |
| Comparative Example 4 | Gum | 0.00222% | | | | |
| Example 1 | Encapsulated retinol: tetrapeptide-1=2.8:1 | 0.00313%+ 0.00109% | | | | |
| Example 2 | Encapsulated retinol: gum=2.8:2 | 0.00313%+ 0.00222% | | | | |

TABLE 2 COL III Detection Scheme

| Group | Sample Name | Dosing Concentration | Stimulation Condition | Detection Model | Detection Item | Detection Method |
|---|---|---|---|---|---|---|
| Blank Control BC | / | / | No stimulation | Fibroblast | COL III (type III collagen) | qRT-PCR |
| Negative Control NC | / | / | UVA (30 J/cm$^2$) stimulation | | | |
| Positive Control PC | TGF-β1 | 100 ng/mL | | | | |
| Comparative Example 1 | Pro-xylane | 0.50% | | | | |
| Comparative Example 2 | Tetrapeptide-1 | 0.00109% | | | | |
| Comparative Example 3 | Encapsulated retinol | 0.00313% | | | | |
| Example 1 | Encapsulated retinol: tetrapeptide-1=2.8:1 | 0.00313%+ 0.00109% | | | | |

3) Dosing: according to the test schemes in TABLE 1 and TABLE 2, when the plating rate of the cells in the 6-well plate reached about 30%-50%, dosing was performed for the groups respectively, with each group in triplicate. The blank control group and the negative control group were added with 2 mL of a culture solution per well, the positive control group was added with 2 mL of a TGF-β1-containing culture solution per well, and sample group was added with 2mL of a test sample-containing culture solution at corresponding concentration per well. After the dosing was completed, the 6-well plate continued to be cultured in the $CO_2$ incubator (37 °C, 5% $CO_2$) for 24 h.

4) UVA stimulation: according to test groups, except the blank control group, other groups were all subjected to UVA irradiation, with an irradiation dosage of 30 J/cm$^2$. After the irradiation was finished, culture was continued in the $CO_2$ incubator (37 °C, 5% $CO_2$) for 24 h.

5) Cell collection: after incubation was ended, an original solution was sucked out and discarded, PBS cleaning was performed twice, and each well was added with 1 mL of RNAiso Plus, to pipet and lyse the cells, after which samples were collected.

6) Detection of gene expression: RNA was extracted and subjected to reverse transcription to cDNA to undergo fluorescence quantitative PCR detection, and result was calculated by a $2^{-\triangle\triangle CT}$ method.

7) Calculation of up-regulation rate:

$$\text{Up-regulation rate (\%)} = \frac{\text{Sample group - Negative control group}}{\text{Negative control group}} \times 100\%$$

8) Data analysis: statistical analysis was performed using t-test method, and normalization processing was performed on amplification factors of mRNA in BC group, where all statistical analysis was double-tailed. Compared with the blank group, significance is expressed by #, $p<0.05$ is expressed by #, $p<0.01$ is expressed by ##; compared with the negative blank group, significance is expressed by*, $p<0.05$ is expressed by *, $p<0.01$ is expressed by **.

See TABLE 3 and TABLE 4 for test results.

TABLE 3 Transcription of Type I Collagen (COL I) of Cell Model

| Group | Raw Material Name | Dosing Concentration | PCR Transcription of Type I Collagen (COL I) | | |
| --- | --- | --- | --- | --- | --- |
| | | | Mean Value | p Value | Up-regulation Rate Compared with NC Group |
| Blank Control BC | / | / | 1 | / | / |
| Negative Control NC | / | / | 0.43 | 0.000## | / |
| Positive Control PC | TGF-β1 | 100 ng/mL | 1.16 | 0.000** | 169.77% |
| Comparative Example 1 | Pro-xylane | 0.5000% | 1.01 | 0.000** | 134.88% |
| Comparative Example 2 | Tetrapeptide-1 | 0.00109% | 0.54 | 0.039** | 25.58% |
| Comparative Example 3 | Encapsulated retinol | 0.00313% | 1.08 | 0.002** | 151.16% |
| Comparative Example 4 | Gum | 0.00222% | 0.46 | 0.598 | 6.98% |
| Example 1 | Encapsulated retinol : tetrapeptide-1=2.8:1 | 0.00313%+ 0.00109% | 1.55 | 0.000** | 260.47% |
| Example 2 | Encapsulated retinol : gum=2.8:2 | 0.00313%+ 0.00222% | 1.2 | 0.000** | 179.07% |

Type I collagen is an extracellular matrix with the largest proportion in the dermis, and has a content of about 85%. Loss of collagen in the dermis is also a main cause of incipient signs of aging, and improving expression of type I collagen is an effective tightening and anti-wrinkle means. TABLE 3 shows that the tetrapeptide-1 and the encapsulated retinol raw material, after being used in combination, remarkably improved the transcription of type I collagen, far superior to using either of the two alone, and also superior to the positive control TGF-β1 and the well-known anti-aging material Pro-xylane. Similar to the tetrapeptide-1, *pistacia lentiscus* gum can also remarkably improve the ability of the encapsulated retinol to stimulate generation of type I collagen, and achieve the effect of synergizing retinol.

TABLE 4 Test Groups of Transcription of Type III Collagen (COL III) based on Cell Model

| | Raw Material Name | Dosing Concentration | PCR Transcription of Type III Collagen (COL III) | | |
|---|---|---|---|---|---|
| | | | Mean Value | p Value | Up-regulation Rate Compared with NC Group |
| Blank Control BC | / | / | 1 | / | / |
| Negative Control NC | / | / | 0.67 | 0.005[##] | / |
| Positive Control PC | TGF-β1 | 100 ng/mL | 1.24 | 0.001** | 85.07% |
| Comparative Example 1 | Pro-xylane | 0.5000% | 0.88 | 0.001** | 31.34% |
| Comparative Example 2 | Tetrapeptide-1 | 0.00109% | 0.71 | 0.195 | 5.97% |
| Comparative Example 3 | Encapsulated retinol | 0.00313% | 0.89 | 0.001** | 32.84% |
| Example 1 | Encapsulated retinol : tetrapeptide-1=2.8:1 | 0.00313%+ 0.00109% | 1.01 | 0.002** | 50.75% |

The type III collagen is an important collagen secondary to the type I collagen in the dermis, and forms collagen fibers with the type I collagen to support dermis structure. TABLE 4 shows that the tetrapeptide-1 and the encapsulated retinol raw material, after being used in combination, remarkably improved the transcription of type III collagen, far superior to using either of the two alone, and also superior to the well-known anti-aging material Pro-xylane.

**Test Example 2 Test of Transcription of Cellular Retinoic Acid Binding Protein 2 (CRABP2) based on Cell Model**

Testing method: based on UVA-stimulated fibroblast model, transcription of CRABP2 was tested using qRT-PCR method, and specific steps were as follows:

1) Cell inoculation: after resuscitation of cells, when a plating rate reached about 60%, the cells were inoculated in a 6-well plate, and incubated overnight in an incubator (37 °C, 5% $CO_2$).

2) Liquid formulation: according to a test scheme (see TABLE 5), test substance working solutions were formulated respectively according to mass ratios.

TABLE 5 CRABP2 Detection Scheme

| Group | Sample Name | Dosing Concentration | Stimulation Condition | Detection Model | Detection Item | Detection Method |
|---|---|---|---|---|---|---|
| Blank Control (BC) | / | / | No stimulation | | | |
| Negative Control (NC) | / | / | | | | |
| Positive Control (PC) | TGF-β1 | 100 ng/mL | | | | |
| Comparative Example 4 | Gum | 0.00222% | | | | |
| Comparative Example 3 | Encapsulated retinol | 0.00313% | UVA (30 J/cm²) stimulation | Fibroblast | CRABP2 cellular retinoic acid binding protein 2 | qRT-PCR |
| Comparative Example 2 | Tetrapeptide-1 | 0.00109% | | | | |
| Example 1 | Encapsulated retinol: tetrapeptide-1=2.8:1 | 0.00313%+ 0.00109% | | | | |
| Example 2 | Encapsulated retinol: gum=2.8:2 | 0.00313%+ 0.00222% | | | | |

3) Dosing: According to the test scheme in TABLE 5, when the plating rate of the cells in the 6-well plate reached about 30%-50%, dosing was performed for the groups respectively, with each group in triplicate. The blank control group and the negative control group were added with 2 mL of a culture solution per well, the positive control group was added with 2 mL of a TGF-β1-containing culture solution per well, and sample group was added with 2 mL of a test sample-containing culture solution at corresponding concentration per well. After the dosing was completed, the 6-well plate continued to be cultured in the $CO_2$ incubator (37 °C, 5% $CO_2$) for 24 h.

4) UVA stimulation: according to test groups, except the blank control group, other groups were all subjected to UVA irradiation, with an irradiation dosage of 30 J/cm². After the irradiation was finished, culture was continued in the $CO_2$ incubator (37 °C, 5% $CO_2$) for 24 h.

5) Cell collection: after incubation was ended, an original solution was sucked out and discarded, PBS cleaning was performed twice, and each well was added with 1 mL of RNAiso Plus, to pipet and lyse the cells, after which samples were collected.

6) Detection of gene expression: RNA was extracted and subjected to reverse transcription to cDNA to undergo fluorescence quantitative PCR detection, and result was calculated by a $2^{-\triangle\triangle CT}$ method.

7) Calculation of up-regulation rate:

$$\text{Up-regulation rate (\%)} = \frac{\text{Sample group} - \text{Negative control group}}{\text{Negative control group}} \times 100\%$$

8) Data analysis: statistical analysis was performed using t-test method, and normalization processing was performed on amplification factors of mRNA in BC group, where all statistical analysis was double-tailed. Compared with the blank group, significance is expressed by #, $p < 0.05$ is expressed by #, and $p < 0.01$ is expressed by ##; compared with the negative blank group; significance is expressed by*, $p < 0.05$ is expressed by *, $p < 0.01$ is expressed by **.

See TABLE 6 for test results.

TABLE 6 Transcription of Cellular Retinoic Acid Binding Protein 2 (CRABP2)

| Group | Raw Material Name | Dosing Concentration | Transcription of Cellular Retinoic Acid Binding Protein 2 (CRABP2) | | |
|---|---|---|---|---|---|
| | | | Mean Value | p Value | Up-regulation Rate Compared with NC Group |
| Blank Control BC | / | / | 1 | / | / |
| Negative Control NC | / | / | 0.48 | 0.000## | / |
| Positive Control PC | TGF-β1 | 100 ng/mL | 0.86 | 0.001** | 79.17% |
| Comparative Example 4 | Gum | 0.00222% | 0.5 | 0.125 | 4.17% |
| Comparative Example 3 | Encapsulated retinol | 0.00313% | 1.14 | 0.000" | 137.50% |
| Comparative Example 2 | Tetrapeptide-1 | 0.00109% | 0.58 | 0.022** | 20.83% |
| Example 1 | Encapsulated retinol : tetrapeptide-1=2.8:1 | 0.00313%+ 0.00109% | 1.34 | 0.000** | 179.17% |
| Example 2 | Encapsulated retinol : gum=2.8:2 | 0.00313%+ 0.00222% | 1.27 | 0.000** | 164.58% |

Cellular retinoic acid binding protein 2 (CRABP2) is a group of intracellular proteins with low relative molecular mass, one of the main physiological functions thereof is to transport retinoic acid (an active form of retinol) into nucleus and activate target gene, so as to regulate and control downstream signaling pathway to play a role in tightening, improving photoaging or the like. TABLE 6 shows that after the *pistacia lentiscus* gum or tetrapeptide-1 and the encapsulated retinol raw material were used in combination, transcription of CRABP2 was remarkably improved, higher than that of using either of the two alone. This means that the *pistacia lentiscus* gum or tetrapeptide-1 can effectively improve intracellular transport efficiency of retinoic acid (an active form of retinol), is suitable as a retinol synergist, and can help the retinol play a better role.

**Test Example 3 Test of 28-day Efficacy on Human Body**

The present test was done in efficacy testing laboratory of Guangzhou Jiyan Cosmetics Technology Co. Ltd. In accordance with the Declaration of Helsinki, selection of volunteers followed ethical standards of human test, and all volunteers had to be tested on their own will, and signed on written informed consent before test.

1) Subjects selection: 10 healthy female volunteers with aging signs on face were chosen with strict adherence to volunteer inclusion criteria, aged between 30 and 60, average age 48.9, maximum age 54, and minimum age 36.

2) Test samples

In the present example, as shown in TABLE 7, a porportion of the encapsulated-retinol composition remained consistent with those in the above cell tests for type I collagen, type III collagen and CRABP2. However, since cells are quite sensitive (inappropriate osmotic pressure/pH will be lethal), dose for cell testing needs to be diluted to a relatively low concentration so as to ensure cell safety. However, during test on human bodies, an active ingredient needs to face difficulties such as ambient pressure and skin barrier, and the active ingredient really entering into skin will be much lower than the dose in formula; therefore, doses of the composition in TABLE 7 are scaled up in a certain proportion. Such scaling up of doses from cell to human body is general knowledge well known to those skilled in the art and is widely used in the development of various raw materials/formulas.

TABLE 7 Formula Table of Test Samples

| Use Purpose | Raw Material Name | Dose % (mass ratio) | Phase |
|---|---|---|---|
| Solvent | Water | 83.9 | A |
| Wetting agent | Glycerin | 5 | A |
| Thickening agent | Hydroxyethyl acrylate/sodium acryloyldimethyl taurate copolymer | 1.8 | A |
| Emulsifier | Polysorbate-60 | 0.1 | B |
| Emulsifier | Sorbitan isostearate | 0.1 | B |
| Emollient | Polydimethylsiloxane | 3 | B |
| Preservative | Phenoxyethanol | 0.3 | C |
| Efficacy composition | Encapsulated retinol: tetrapeptide-1 : gum =2.8:1:2 | 5.8 | D |

Preparation method: phase-A raw materials were accurately weighed, stirred and mixed uniformly (250 rpm × 30 min), all phase-B raw materials were added, mixture was homogenized using a high-speed homogenizer for 5 min at 8000 rpm, to render a uniformly mixed emulsion, which was stirred for 30 min at 300 rpm, during which phase-C and phase-D raw materials were added, and then resultant was filled in a vacuum emulsion vial and stored in a sealed manner.

1) Use method: during test, the volunteers were required to use test samples on the entire face, once a day at night. After face washing, a suitable amount of product was smeared to the face, gently massaged until absorbed, continuously for 28 days. During test, the subjects should strictly follow the use method, without exposure to the sun or doing medical cosmetology, and keeping regular schedule, so as to avoid interference with test results.

2) Test period: 28 days. The subjects were visited and detected three times before being included in the test to use product (baseline value D0), 14 days after using product (D14) and 28 days after using product (D28).

3) Test environmental conditions: at each visit, the subjects needed to wash face first, and then sit still for 30 min under environmental conditions of temperature of (21±1)°C and relative humidity of (50±10)%, and then data were acquired using instrument.

4) Test parameters:

TABLE 8 Table of Human Body Test Parameters

| **Skin Elasticity** | |
|---|---|
| Tested Part | One cheek (random) |
| Instrument | ElastiMeter skin elasticity tester |
| Testing Method | Testing 3 times, averaging |
| Testing Time Point | Before using product (D0), 14 days (D14) and 28 days (D28) after using product |
| Parameter Description | Increased test value indicating better skin elasticity |
| **Skin Brightness L\*Value** | |
| Tested Part | One cheek (random) |
| Instrument | VISIA7+IPP analysis |
| Testing Method | VISIA7 photographing once, IPP analyzing L value |
| Testing Time Point | Before using product (D0), 14 days (D14) and 28 days (D28) after using product |
| Parameter Description | Increased test value indicating brighter skin |

5) Data analysis: mean values of various parameters at various time points were calculated for tested product group, and differences between values after using the product (D14/D28) and values before using the product (D0) were respectively calculated. Rate of change = [(after use - before use) / before use] * 100%. SPSS software was applied to perform normal distribution detection on the above differences, and pair T test was used to analyze differences between data. When the data were not in normal distribution, the differences between the data were analyzed using rank sum test. If statistical significance was $p<0.05$, it indicated that the test product group had statistically significant difference between before and after using the product; and if the statistical significance was $p\geq0.05$, it indicated that the test product group had no statistically significant difference before and after using product.

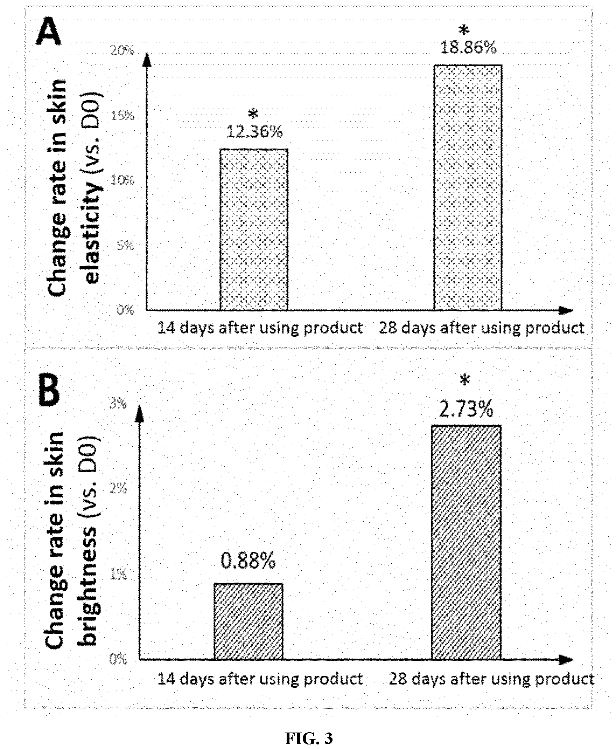

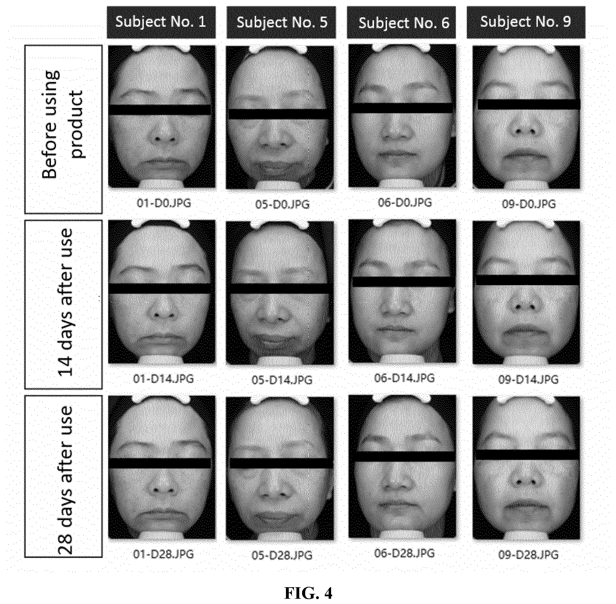

See FIG. 3, FIG. 4 and TABLE. 9 for test results. FIG. 3 shows changes in the skin elasticity (A) and skin brightness L value (B) after the subjects used cosmetics containing the encapsulated-retinol composition, where * represents $p<0.05$. FIG. 4 shows photographs of faces of the subjects taken by Visia.

TABLE 9 Changes in Skin Elasticity and Skin Brightness L Value after Subjects Using Cosmetic Containing Encapsulated-retinol Composition

| Parameter | Mean Value | | | Change rate (vs. before use) | | | |
|---|---|---|---|---|---|---|---|
| | Before using product (D0) | 14 days after using product (D14) | 28 days after using product (D28) | 14 days after using product (D14) | | 28 days after using product (D28) | |
| | | | | Change rate | p value | Change rate | p value |
| Skin Elasticity | 41.53 | 46.67 | 49.37 | 12.36% | 0.045* | 18.86% | 0.001* |
| Skin Brightness L *Value | 66.88 | 67.47 | 68.71 | 0.88% | 0.266 | 2.73% | 0.018* |

During aging, skin elasticity decreases and dullness increases, which are the most typical signs of aging. FIG. 3 shows that after the subjects used skin care products containing encapsulated retinol, tetrapeptide-1 and gum for 14 days as required, the skin elasticity was remarkably improved by +12.36% ($p<0.05$, having significance); after 28 days, the skin elasticity was remarkably improved by +18.86% ($p<0.05$, having significance), and the skin brightness L value was increased by +2.73% ($p<0.05$, having significance), which means that long-term use of the composition of encapsulated retinol, tetrapeptide-1 and gum can effectively improve the skin elasticity and brightness of the subjects and improve the aging signs of facial skin.

The above-mentioned are merely for preferred embodiments of the present disclosure. It should be noted that for those ordinarily skilled in the art, various improvements and modifications also could be made to without departing from the principle of the present disclosure, and the improvements and modifications also should be regarded as the scope of protection of the present disclosure.

**Claims**

1.   A composition, comprising encapsulated retinol and a synergist, wherein

the synergist comprises one or more of tetrapeptide-1 or *pistacia lentiscus* gum; and
a mass ratio of the encapsulated retinol to the synergist is 2-3:1-2.

2.   The composition according to claim 1, wherein a mass ratio of the encapsulated retinol to the synergist is 2.5-3:1-2.

3.   The composition according to claim 1 or 2, wherein the encapsulated retinol comprises retinol and a shell encapsulating the retinol, wherein
the shell is formed from cellulose acetate butyrate, tricaprylin, pentaerythrityl tetra-di-t-butyl hydroxyhydrocinnamate, silica silylate, silica and magnesium stearate.

4.   The composition according to claim 1, wherein trade name of the encapsulated retinol is CelluCap RL; and trade name of the tetrapeptide-1 is Uplevity e-Lift peptide.

5.   The composition according to claim 4, wherein a mass ratio of the encapsulated retinol to the synergist is 2.5-3:1.

6.   The composition according to claim 5, wherein the mass ratio of the encapsulated retinol to the synergist is 2.7-3:1.

7.   The composition according to claim 6, wherein the mass ratio of the encapsulated retinol to the synergist is 2.7-2.8:1.

8.   Use of the composition according to any one of claims 1 to 7 in preparation of anti-aging products.

**9.** The use according to claim 8, wherein the products comprise cosmetics.

**10.** A product, comprising the composition according to any one of claims 1 to 7.

**FIG. 1**

**FIG. 2**

**FIG. 3**

**FIG. 4**

**Europäisches Patentamt**
**European Patent Office**
**Office européen des brevets**

# EUROPEAN SEARCH REPORT

**Application Number**

EP 24 22 0383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 26 September 2017 (2017-09-26), anonymous: "Élixir Perfect Lifting Essence", XP055505495, Database accession no. 5094753 | 1,2,4-10 | INV. A61K8/11 A61K8/64 A61K8/9789 A61Q19/08 A61K8/67 |
| Y | * abstract * ----- | 3 | |
| Y | CN 114 869 798 B (GUANGDONG MARUBI BIOLOGICAL TECH CO LTD) 21 November 2023 (2023-11-21) * paragraphs 1-4,7,14,47 - pages 10-15 * ----- | 1-10 | |
| Y | CN 108 451 797 A (GUANGZHOU SALIAI STEMCELL SCIENCE & TECHNOLOGY CO LTD) 28 August 2018 (2018-08-28) * paragraphs [0060], [0063], [0077] - [0079], [0084] * ----- | 1-10 | |
| Y | US 2010/240628 A1 (JENKINS GAIL [GB] ET AL) 23 September 2010 (2010-09-23) * paragraphs [0087], [0091]; claims 13-15 * ----- | 1-10 | **TECHNICAL FIELDS SEARCHED (IPC)** A61K A61Q |

The present search report has been drawn up for all claims

| Place of search | Date of completion of the search | Examiner |
|---|---|---|
| Munich | 19 May 2025 | Tironi, Matteo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 22 0383

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2025

| Patent document cited in search report | | Publication date | Patent family member(s) | | Publication date |
|---|---|---|---|---|---|
| CN 114869798 | B | 21-11-2023 | CN | 114869798 A | 09-08-2022 |
| | | | JP | 2023169857 A | 30-11-2023 |
| CN 108451797 | A | 28-08-2018 | NONE | | |
| US 2010240628 | A1 | 23-09-2010 | EP | 1624853 A2 | 15-02-2006 |
| | | | US | 2007010501 A1 | 11-01-2007 |
| | | | US | 2010240628 A1 | 23-09-2010 |
| | | | WO | 2004103320 A2 | 02-12-2004 |

EPO FORM P0459

For more details about this annex : see Official Journal of the European Patent Office, No. 12/82